# EUROPEAN PATENT SPECIFICATION

(11) **EP 1 573 017 B1**
(45) Date of publication and mention of the grant of the patent: **09.07.2008**
(21) Application number: 03758088.3
(22) Date of filing: 03.11.2003
(51) Int. Cl.: C12P 1/02, C07K 14/46

(54) **PREPARATION OF ANTIFREEZE PROTEIN**
HERSTELLUNG VON FROSTSCHUTZPROTEIN
PREPARATION D'UNE PROTEINE ANTIGEL

(30) Priority: 20.12.2002 EP 02258921
(43) Date of publication of application: 14.09.2005
(73) Proprietor: UNILEVER PLC, London EC4P 4BQ (GB); UNILEVER N.V., 3013 AL Rotterdam (NL)
(72) Inventor: CHAPMAN, John William Unilever R & D Vlaardingen, NL-3133 AT Vlaardingen (NL); VAN DE LAAR, Teun Unilever R & D Vlaardingen, NL-3133 AT Vlaardingen (NL); LINDNER, Nigel Malcolm Unilever R & D Colworth, Bedford, Bedfordshire MK44 1LQ (GB); VISSER, Christiaan Unilever R & D Vlaardingen, NL-3133 AT Vlaardingen (NL)
(74) Representative: Hugot, Alain
(86) International application number: PCT/EP2003/012219
(87) International publication number: WO 2004/057007

(56) References cited:
- WO-A-94/04687
- WO-A-97/02343
- WO-A-99/37673
- WO-A-99/37782
- CHAO H ET AL: "STRUCTURE-FUNCTION RELATIONSHIP IN THE GLOBULAR TYPE III ANTIFREEZE PROTEIN: IDENTIFICATION OF A CLUSTER OF SURFACE RESIDUES REQUIRED FOR BINDING TO ICE" PROTEIN SCIENCE, CAMBRIDGE UNIVERSITY PRESS, CAMBRIDGE, GB, vol. 3, 1994, pages 1760-1769, XP000601182 ISSN: 0961-8368

## Description

### Background to the invention

Antifreeze proteins (AFPs) are polypeptides produced by a wide range of species, particularly those indigenous to colder climes, which have the ability to inhibit freezing of water and aqueous materials at temperatures below 0°C. In general, it is thought that these proteins function by means of interacting with and inhibiting the growth of ice crystals, but it is now clear that there are different classes of antifreeze protein which may have different mechanisms of action and different effects. For example, in addition to causing a thermal hysteresis in the freezing/melting behaviour of ice/water systems, AFPs can influence the shape and size of the crystals of ice formed when freezing does occur, and inhibit recrystallisation of ice. More recently, it has been suggested that these proteins should instead be known as Ice Structuring Proteins (ISPs) (Clarke, C.J., Buckley, S.L., and Lindner, N., Cryoletters., 23 (2002) 89-92)

These attributes of AFPs mean that they can have profound effects on properties such as the ease of production, the texture and the stability during storage of various frozen preparations and, in recent years, there has been much interest in their possible commercial application, especially in the food industry. For example, control of ice crystal dimensions can lead to particularly favourable textures in frozen confections. Improvements in storage properties also result from the inclusion of AFPs in the formulation. A review of the occurrence of AFPs and their potential use in the food industry has been presented by Griffith and Vanya Ewart in Biotechnology Advances, vol 13, pp. 375-402 (1995).

To be well suited to such a purpose an AFP needs to combine desirable effects on the frozen materials in which it incorporated, and to be readily prepared on an industrial scale. The latter requirement can be particularly problematic since many of the species in which AFPs have been identified are not readily amenable to commercial harvesting or processing. Some AFPs have been found to be very susceptible to denaturation, which places severe constraints on the isolation methods which can be applied to them. In view of these difficulties, there has been considerable interest in the production of AFPs by means of expressing cloned genes encoding them in more convenient expression hosts, such as microorganisms or easily cultivated and processed plants. For many AFPs, however, this has proved problematic: they are often obtained in poor yield and sometimes lacking in activity.

Among the most potentially useful AFPs which have been identified is a type III AFP from the Ocean Pout, which has been designated HPLC-12. This protein was found to excel in its ability to aid in controlling the shape and size of ice crystals. The protein was shown, for example, to outperform the well-known type I AFPs in recrystallisation tests. A further advantageous property identified for type III HPLC-12 was that although it was not produced in substantial amounts in E. coli, it could be produced in good yield by expression of a cloned gene encoding its sequence in a transformed yeast, thus providing a potentially much more convenient and economically viable source for industrial scale production than the fish in which the protein naturally occurs.

Protein glycosylation involves a large number of enzymes and deficiencies in one or more of these can potentially alter the pattern of glycosylation. A loss of activity of the enzyme responsible for transferring the first sugar residue onto a protein could potentially prevent any glycosylation. Accordingly, use of such a protein mannosyl transferase (pmt)-deficient mutant yeast strain has been suggested as a way to overcome the problem of abnormal glycosylation of heterologously expressed proteins (WO 94/04687). However the situation is complicated by the fact that there is not just one enzyme with this function but several, with different protein-specificities. For example, in a review on protein O-mannosylation, Strahl-Bolsinger et al (Biochimica et Biophysica Acta 1426 (1999) 297-307) noted that of ten O-glycosylated proteins studied, six are glycosylated in yeast by the enzymes Pmt1 and Pmt2, while the other four show a decrease or lack of O-mannosylation exclusively in strains where the activity of the enzyme Pmt4 has been abolished. None of the analysed proteins was seen to be hypoglycosylated in another class of mutant, in which the activity of enzyme Pmt3 is lost, however this mutation did result in reduced O-mannosylation of chitinase in the genetic background of a *pmtlpmt2* double mutation. No correlation between mannosylation specificity and any sequence or structural features of the protein substrate has been identified, so it is not possible to predict which particular transferase enzyme(s) are likely to be responsible for initiating the glycosylation of any particular protein, whether it is a native protein of the glycosylating species, or a foreign protein produced by heterologous expression therein.

### Summary of the invention

The present inventors have now found, however, that type III HPLC-12 produced in yeast has a specific activity, as measured in a recrystallisation inhibition assay, that is lower than that of the protein isolated from Ocean Pout blood. They have been able to show that this is a consequence of O-glycosylation of the protein by the yeast, which does not occur when the native protein is produced by the fish. Surprisingly, only the non-glycosylated species is active.

This was unexpected since such experimental evidence as there is relating to AFPs reveals no clear and consistent pattern with respect to glycosylation and functionality: indeed some AFPs are naturally extensively glycosylated. For example, DeVries et al (Science 172 (1971) 1152), reported that the activity of an AFP found in northern cods and Antarctic notothenioids loses its activity if the pendant disaccharides are removed. In other cases, the glycosylation that occurs in nature has been shown not to be important for the AFP activity. For example, Worrall et al (Science 282 (1998)115-117), found that when a naturally glycosylated AFP from carrots was produced without its surface glycans, its recrystallisation inhibition activity was unaffected. A similar lack of dependence on glycosylation, even though it is naturally present, for AFP activity has been noted by the present inventors in the case of a heterologously expressed AFP from rye grass. Thus there is no clear indication of a general link between glycosylation and activity among AFPs.

Further, as a consequence of this unexpected finding, the present inventors have been able to devise a method for substantially suppressing this abnormal glycosylation of type III AFP, and thereby have provided a means for producing type III AFPs, such as type III HPLC-12 protein which combines the convenience and cost-effectiveness of yeast as a host organism, whilst yielding a product with a potency approaching that of the native protein. The present inventors have found that using this method, both the yield of recombinant protein and the specific activity of the -recombinant protein can be increased, i.e. more protein can be recovered from the host cells, and of that protein, a greater proportion of the protein is active.

Accordingly, in a first aspect, the present invention provides a method for producing a type III antifreeze protein (AFP) which method comprises expressing in a fungal host cell which is deficient in glycosylation of type III AFP in comparison to the parent strain, a nucleic acid sequence encoding the AFP, wherein the fungal host cell is a yeast which is a pmt1-deficient and /or a pmt2-deficient mutant strain.

In a preferred embodiment, the yeast is *Saccharomyces cerevisiae.*

In a related aspect, the present invention also provides a method for increasing the specific antifreeze activity of an antifreeze protein (AFP) type III, or a functional equivalent thereof, when said protein is prepared by expression in a heterologous fungal species of a gene/nucleic acid sequence encoding the protein, by means of reducing the extent of glycosylation of the protein.

Preferably the specific AFP activity is measured by means of an ice recrystallisation inhibition assay.

In a preferred embodiment, the reduction in protein glycosylation is achieved by means of selecting a strain of the expressing species which is deficient in the activity of one or more enzymes involved in protein glycosylation, preferably one or more protein mannosyl transferase enzymes.

A suitable glycosylation-deficient strain is typically selected from a range of such strains by analysis of the AFP type III protein which is produced when a gene encoding said protein is expressed in said strains. Preferably, the analysis of the AFP type III protein is based on an assay of its AFP activity or functionality, more preferably its ice recrystallisation inhibitory activity.

In a second aspect, the present invention provides a composition comprising recombinant type III antifreeze protein (AFP) wherein from about 50% to 99% of the AFP is unglycosylated. Preferably, the type III AFP is type III HPLC-12.

In a preferred embodiment, the composition is obtainable, more preferably obtained, by the method of the first aspect of the invention.

In a third aspect, the present invention provides a method for identifying a fungal host strain capable of expressing a type III AFP such that less than about 50% of the expressed AFP is glycosylated, which method comprises:
(i) providing a plurality of fungal host cells comprising a nucleic acid sequence which directs expression of the AFP in the host cell;
(ii) culturing the host cells under conditions that allow for expression of the AFP; and
(iii) determining the extent of glycosylation of the expressed AFP.

In a preferred embodiment, the plurality of host cells have been subjected to a mutagenesis step.

### Detailed description of the invention

This invention is based upon the finding that when the antifreeze protein type III HPLC-12 is prepared by expression in a normal yeast strain of a nucleic acid sequence encoding the protein, a substantial proportion of the secreted protein product has been glycosylated. Such glycosylation is not present in the native protein, and recrystallisation inhibition assays on the separated glycosylated and unglycosylated fractions showed, surprisingly, that the glycosylation effectively abolished the AFP activity of the protein.

In view of this surprising finding, the inventors have gone on to devise a method for increasing the specific activity of the type III HPLC-12 antifreeze protein, or a functional equivalent thereof, when said protein is prepared by expression in a heterologous fungal species of a nucleic acid sequence encoding the protein sequence, by means of reducing the extent of glycosylation of the protein.

Unless defined otherwise, all technical and scientific terms used herein have the same meaning as commonly understood by one of ordinary skill in the art (e.g. in cell culture, molecular genetics, nucleic acid chemistry, hybridisation techniques and biochemistry). Standard techniques are used for molecular, genetic and biochemical methods (see generally, Sambrook et al., Molecular Cloning: A Laboratory Manual, 3rd ed. (2001) Cold Spring Harbor Laboratory Press, Cold Spring Harbor, N.Y. and Ausubel et al., Short Protocols in Molecular Biology (1999) 4th Ed, John Wiley & Sons, Inc. - and the full version entitled Current Protocols in Molecular Biology, and chemical methods.

### Antifreeze proteins

For the purposes of this invention, an antifreeze protein is a protein which has significant ice recrystallisation inhibition properties and is therefore an ice structuring protein (ISP). Ice recrystallisation inhibition properties can conveniently be measured by means of a modified splat assay, as described in WO 00/53029. Significant ice recrystallisation inhibitory activity can be defined as where a 0.01 wt% solution of the AFP in 30 wt% sucrose, cooled rapidly (at least Δ50°C per minute) to -40°C, heated rapidly (at least Δ50°C per minute) to -6°C and then held at this temperature results in an increase in average ice crystal size over one hour of less than 5 µm. The specific activity is a measure, per unit concentration of the dissolved AFP, of this ability of the protein to limit the extent of increase in size of ice crystals as a result of recrystallisation, in a given time.

The antifreeze proteins according to the present invention are type III AFPs. These AFPs have to date been identified in a number of polar fish of the family Zoarcidae such as *Macrozoarces americanus* (Eel pout, Ocean pout) and Anarhichas lupus (Wolf fish) - Barrett, 2001, Int. J. Biochem. Cell Biol. 33: 105-117. Type III AFPs typically have a molecular weight of from about 6.5 to about 14 kDa, a beta sandwich secondary structure and a globular tertiary structure.

A number of genes encoding type III AFPs have been cloned (Davies and Hew, 1990, FASEB J. 4: 2460-2468). A particularly preferred type III AFP is type III HPLC-12.

The amino acid sequence of Ocean pout type III HPLC-12 is shown as SEQ ID NO:1. Type III HPLC-12 polypeptides according to the present invention include polypeptides having the amino acid sequence shown as SEQ ID NO:1 and functional equivalents thereof.

By "functional equivalent" is meant any polypeptide whose sequence has at least 80%, more preferably at least 85%, 90% or 95% sequence identity with the sequence of type III HPLC-12 as shown in SEQ ID NO: 1 and which exhibits AFP activity, in particular ice recrystallisation inhibitory (RI) activity. It is preferred that functional equivalents have at least 50% of the RI activity of a polypeptide having the amino acid sequence of type III HPLC-12 as shown in SEQ ID No:1, more preferably at least 60%, 70% or 80% of the RI activity of a polypeptide having the amino acid sequence of type III HPLC-12 as shown in SEQ ID No:1. RI activity can be conveniently be measured by means of a modified splat assay, as described in WO 00/53029.

Sequence identity calculations are typically performed with the aid of readily available sequence comparison programs. These commercially available computer programs can calculate % homology, typically % identity, between two or more sequences.

Most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel *et al*., 1999 *ibid* - Chapter 18), FASTA (Atschul et al., 1990, J. Mol. Biol., 403-410) and the GENEWORKS suite of comparison tools. Both BLAST and FASTA are available for offline and online searching (see Ausubel *et al*., 1999 *ibid*, pages 7-58 to 7-60). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

In a highly preferred embodiment, the AFP polypeptide is linked to a signal sequence that directs secretion of the type III AFP protein from the host cell. Suitable signal sequences include the *S*. *cerevisiae* invertase signal sequence and the pre-sequence of the α-mating factor of *S*. *cerevisiae.*

The AFP may be fused to a heterologous sequence to form a fusion protein, to aid in extraction and purification. Examples of fusion protein partners include glutathione-S-transferase (GST), hexahistidine, GAL4 (DNA binding and/or transcriptional activation domains) and β-galactosidase. It may also be convenient to include a proteolytic cleavage site between the fusion protein partner and the protein sequence of interest to allow removal of fusion protein sequences. Preferably the fusion protein will not hinder the RI activity of the AFP. However, for the production of AFPs for use in the preparation of foodstuffs, it is preferable to avoid the use of fusion partners.

### AFP-encoding nucleic acids and expression vectors

The method of the invention comprises expressing type III AFPs in a glycosylation-deficient fungal host. This is achieved by introducing into the fungal host a nucleic acid sequence, typically an expression vector which encodes the type III AFP, together with sequences required for directing expression of the type III AFP in the host cell. Thus, nucleic acid sequences encoding a type III AFP are generally incorporated into a recombinant replicable nucleic acid vector suitable for introduction into the fungal host cell. A nucleic acid sequence encoding a type III AFP may, for example, be a cDNA sequence, a genomic DNA sequence, a hybrid DNA sequence, or a synthetic or semi-synthetic DNA sequence. It is preferred to use a cDNA rather than a genomic DNA.

The nucleic acid sequence encoding the type III AFP is operably linked to a control sequence that is capable of providing for the expression of the coding sequence by the host cell, i.e. the vector is an expression vector. The term "operably linked" means that the components described are in a relationship permitting them to function in their intended manner. A regulatory sequence "operably linked" to a coding sequence is ligated in such a way that expression of the coding sequence is achieved under conditions compatible with the control sequences.

The control sequences will include sequences such as promoters, and optionally transcriptional enhancer elements. Preferably, the promoter is a strong promoter such as a GAPDH promoter of *S*. *cerevisiae* or the GAL7 promoter. Promoters may be constitutive, such as the GAPDH promoter or inducible, such as the GAL7 promoter.

The nucleic vector is transformed into a suitable fungal host cell using standard techniques such as heat shock or electroporation, to provide for expression of the type III AFP. This process may comprise culturing a host cell transformed with an expression vector as described above under conditions to provide for expression by the vector of the coding sequence encoding the type III AFP, and optionally recovering the expressed protein.

A suitable method for expression of a type III HPLC-12 polypeptide in S. *cerevisiae* is described in WO 97/02343, except that the host cell will be glycosylation-deficient as described below.

Where the type III AFP is not linked to a signal sequence, the protein can be recovered from the host cells by standard techniques such as lysing the cells and purifying the recombinant protein from the cell lysate. Where a signal sequence is used, the protein can be recovered from the culture supernatant.

### Fungal host cells with reduced glycosylation

A reduction in the glycosylation of the AFP is typically achieved by inhibiting or abolishing the activity of gene products, such as enzymes, involved in the host cell glycosylation pathways. Preferably, said glycosylation is O-glycosylation, by which is meant attachment of pendant carbohydrate moieties to serine and/or threonine residues at the protein surface.

In a preferred embodiment the reduction in glycosylation is achieved by means of selecting a strain of the expressing organism which is deficient in the activity of one or more enzymes involved in protein glycosylation. Preferably said enzyme is one involved in the attachment of a sugar residue directly to an amino acid side chain of the protein substrate. More preferred is an enzyme involved in the attachment of a mannosyl residue to the hydroxyl group of a serine or threonine residue of the protein substrate (O-glycosylation). Because there are typically several such enzymes active in a given fungal strain, it is necessary to select strains deficient in the activity of the specific enzymes that are effective in glycosylating type III HPLC-12 specifically.

The host strain is typically deficient in the activity of an enzyme of interest due to one or more mutations in the corresponding gene. The mutation may be in the coding sequence, such as an insertion, deletion or substitution affecting the activity, conformation and/or stability of the resulting polypeptide. The mutation may also be in the regulatory control sequences, such as the promoter and/or 5'UTR, leading to a reduction in expression of the gene product. However, it is also possible that the activity of an enzyme of interest is affected by a mutation in another gene product which interacts with the enzyme of interest.

Typically, a suitable strain for expression is selected from among glycosylation-deficient mutant strains which have already been identified for the species in which expression is to be carried out. In the case of expression in *S*. *cerevisiae,* for example, at least four genes have been identified which encode proteins involved in transfer of a mannosyl residue to protein serine or threonine residues, said genes being designated pmt1, pmt2, pmt3 and pmt4. The present inventors were able to investigate mutants in which the activity of one or more of these genes was known to be disrupted. They were thus able to determine that disruption of either pmt1 or pmt2 was effective in reducing the extent of glycosylation of secreted type III HPLC-12. The yield of secreted protein was also found to be affected by the mutations and it was found that the most preferred gene disruption for the purposes of this invention is that of pmt1, since this produces the highest yield of the unglycosylated, active type III HPLC-12 protein. By contrast, disruption of the gene pmt4 did not have an appreciable effect on the extent of glycosylation or yield.

Accordingly, the invention provides a method for preparing type III HPLC-12, or a functional equivalent thereof, with enhanced specific AFP activity (in comparison to that obtained when the protein is produced in the parent strain) by means of expressing a nucleotide sequence encoding said protein in a fungal host strain deficient in the activity of the enzymes encoded by one or more of the genes pmt1 and pmt2, or homologues thereof. Preferably, the gene disrupted is pmt1.

In this context, the term "homologue" thereof means a gene which encodes a gene product having the same function as pmt1 or pmt2 gene products.

In a preferred embodiment, the fungal host cell is an *Saccharomyces cerevisiae* cell with a disrupted pmt1 gene and/or a disrupted pmt2 gene.

The extent of glycosylation of type III HPLC-12 produced by any chosen expressing strain can be readily gauged by methods that are sensitive to the increased molecular weight of the modified protein. For example, the additional mass of the glycan attachments is readily apparent in SDS-PAGE. The application of this method can further be aided by using an antibody preparation, specific for HPLC-12, to perform a Western blot in which the bands due to the AFP (glycosylated and unglycosylated) are specifically detected, with the background of other proteins suppressed. This allows the identification of strains which are effective in suppressing HPLC-12 glycosylation to be identified, for example, without the need to purify the HPLC-12 protein from the medium into which it is secreted. Suitable monoclonal or polyclonal antibodies can readily be prepared by conventional methods. Alternatively, the level of glycosylated and non-glycosylated type III HPLC-12 can be determined using reverse phase HPLC. Furthermore, the HPLC system coupled to mass spectroscopy can be used to investigate specific glycoforms.

Methods such as SDS gel electrophoresis, Western blot, HPLC analysis and HPLC coupled to mass spectroscopy were used by the present inventors to identify the preferred strains of *S. cerevisiae* for type III HPLC-12 production. It could readily be extended to investigate further mutant strains of this or other species, in order to seek out still more effective expression hosts. Alternatively, an assay based on the recrystallisation inhibition properties of an at least partially purified type III HPLC-12 containing preparation could be used to detect conditions or strains that produce a good yield of active protein.

By identifying the absence of glycosylation as the key criterion in assessing the utility of the product as an antifreeze protein, and by thus providing convenient methods to assay this, the inventors have thus provided a general method for identifying fungal strains suited to the production of active type III HPLC-12 in good yield.

In *S. cerevisiae* there are a number of other genes which have been identified, whose expression products are enzymes involved in later stages of glycosylation and which, in some cases are involved in both O- and N-linked glycosylation (Strahl-Bolsinger et al (Biochimica et Biophysica Acta 1426 (1999) 297-307)). Mutants in which these genes have been disrupted could also, in principle be investigated to gauge their suitability for the production of type III HPLC-12 or a functional equivalent.

The method could also readily be extended to other species. In some cases glycosylation-deficient mutants have already been described and in other cases these could be readily identified. For example, WO 94/04687 describes the cloning of a homologue of pmt1 from another yeast, *Kluyveromyces lactis.* This was readily achieved by PCR, using primers designed using sequence information from the *Saccharomyces* gene. The authors go on to describe how sequencing of the *Kluyveromyces lactis* gene would allow a disruption mutant to be constructed for this species. The same strategy could straightforwardly be applied to other fungi. In the light of the finding by the present inventors that the enzymes encoded by pmt1 and pmt2 are most effective in glycosylating type III HPLC-12 in *Saccharomyces,* it is probable that proteins homologous to these would be suitable targets for disruption in other species. Once suitable candidate glycosylation mutants are thus acquired or, if necessary, constructed for other fungal species, the same strategy that has been exemplified by the authors for *Saccharomyces* would be applicable to identify the strain in which the best yield of active type III HPLC-12 is obtainable.

In one embodiment, suitable glycosylation-deficient host cells can be obtained by subjecting a population of host cells to mutagenesis to obtain a population of mutant host cells and then screening the population of cells as described above for cells that are defective in protein glycosylation, in particular glycosylation of a type III AFP. Mutagenesis of cells can be carried out using standard techniques such as random mutagenesis, using for example DNA-damaging chemicals or UV/X-ray irradiation, or site-directed mutagenesis, using for example primers directed to known pmt genes in one fungal species to target a homologous sequence in another species).

The species in which expression is carried out may be any suitable fungal species, encompassing yeasts such as (but not limited to) those of the genera *Saccharomyces, Kluyveromyces, Pichia, Hansenula, Candida, Schizosaccharomyces* and the like, and filamentous species such as (but not limited to) those of the genera *Aspergillus, Trichoderma, Mucor, Neurospora, Fusarium* and the like. Preferably the species selected is a yeast, most preferably a species of *Saccharomyces* such as S. *cerevisiae.* Abnormal glycosylation has been shown to be a feature of expression of heterologous genes in many of these genera.

However, in an alternative embodiment, it may be desirable to use a fungal host strain of a species that does not naturally carry out significant O-glycosylation. Thus the term "glycosylation-deficient" in the context of the present invention is not limited to host cells that have been genetically manipulated to reduce protein glycosylation. Nonetheless, typically, a glycosylation-deficient host cell is one which comprises mutations in one or more genes involved in protein glycosylation.

### AFP compositions

The method of the invention makes it possible to obtain highly active preparations of type III AFPs containing a high proportion of unglycosylated AFP.

Thus the present invention provides a composition comprising at least about 50% by weight of unglycosylated type III AFP calculated as a percentage of total type III AFP, obtainable by expression of a nucleic acid sequence encoding the type III AFP in a glycosylation-deficient fungal host cell. Preferably, the composition comprises at least about 60%, 65%, 70% or 80% by weight of the unglycosylated type III AFP calculated as a percentage of total type III AFP.

Accordingly, the composition of the invention also comprises less than about 50% by weight of glycosylated type III AFP, calculated as a percentage of total type III AFP, more preferably less than about 40, 35, 30 or 20%. Alternatively expressed, a composition of the invention comprises unglycosylated type III AFP and glycosylated type III AFP in a weight ratio of from 1:1 to 100:1, more preferably from 1.5:1 to 100:1, most preferably from 2:1, 3:1 or 4:1 to 100:1. Since the type III AFP is expressed in a fungal host, rather than a prokaryotic host, there will generally be at least trace amounts of glycosylated type III AFP, which would not be present in type III AFP expressed in a prokaryotic host cell.

In a preferred embodiment, the composition of the invention comprises from 50 to 99% by weight of type III AFP lacking O-glycosylation.

In addition, it is preferred that the composition is at least 30% pure with respect to type III AFP (regardless of glycosylation type) based on total protein content, more preferably at least 40, 50 or 60% pure. Where the AFP is to used for cosmetic or pharmaceutical applications then it is preferred that the AFP is at least 90% pure with respect to type III AFP (regardless of glycosylation type) based on total protein content, more preferably at least 95,98 or 99% pure.

The present invention will now be described with reference to the following examples which are illustrative only and non-limiting. The examples refer to figures:

### Description of the figures

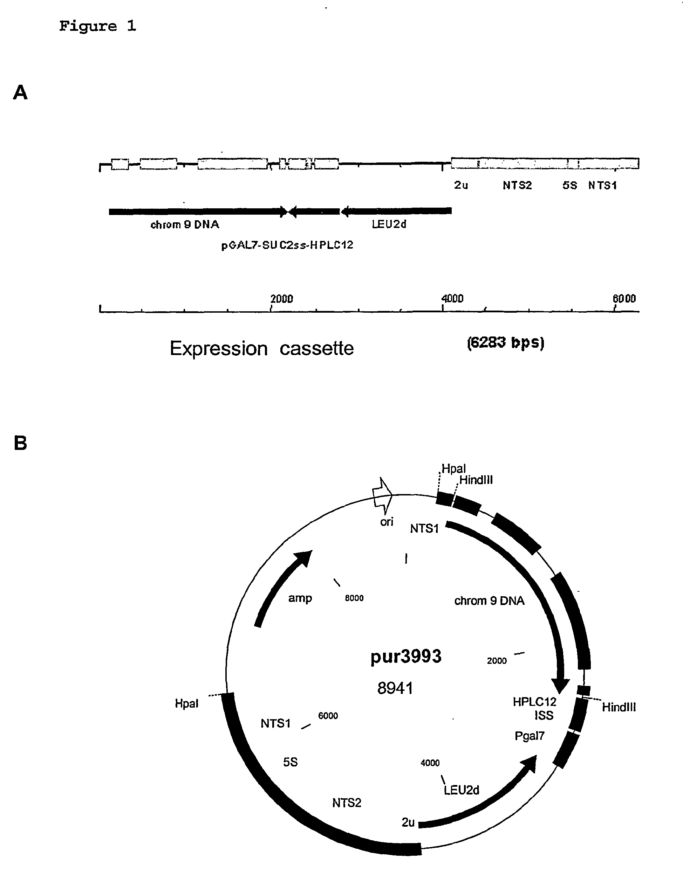

Figure 1a is a schematic representation of the rDNA integration cassette used in the examples. The cassette contains the following sequences:

| | |
|---|---|
| 1-126 | NTS1 - *S*. *cerevisiae* rDNA non transcribed spacer |
| 127-2186 | *S*. *cerevisiae* chromosome IX DNA |
| 114-348 | partial orf1= hypothetical protein |
| 485-916 | RNAse P subunit |
| 1165-1959 | weak similarity. to glucosidase, exo sialidase, mucins |
| 2103-2165 | questionable orf |
| 2165-2096 | transcriptional activator of sulfur a.a. metabolism |
| 2397-2197 | Antifreeze protein type III HPLC12 |
| 2457-2398 | ISS - *S*. *cerevisiae SUC2* I(Invertase) signal sequence |
| 2775-2486 | Pgal7 - *S*. *cerevisiae* GAL7 promoter (synthetic) |
| 4009-2801 | LEU2d - *S*. *cerevisiae LEU2d* |
| 4100-4413 | 2u - *S*. *cerevisiae* 2u plasmid fragment (non functional) |
| 4420-5460 | NTS2 - *S*. *cerevisiae* rDNA non transcribed spacer |
| 55461-5581 | 5S - *S*. *cerevisiae* rDNA 5S RNA |
| 5582-6238 | NTS1 - *S*. *cerevisiae* rDNA non transcribed spacer |

Figure 1b is a schematic representation of plasmid pUR3993.
Figure 2 shows an HPLC chromatogram.

### Examples

### Example 1: Determination of recrystallisation inhibition activity of glycosylated and unglycosylated forms of AFP type III HPLC-12 produced by Saccharomyces cerevisiae

Enriched fractions of glycosylated and non-glycosylated AFP type III were prepared from fermentation broth.

Fermentation broth containing AFP type III HPLC-12 (15ml) was pipetted into separate conical tubes and 10ml refrigerated ethanol added and mixed for 5 seconds.

Where the pH was lower than 6.0, the pH was corrected with 1M NaOH. The tube was then left on ice for at least 20 minutes or overnight in the freezer before being centrifuged at a temperature of 5°C for 5 minutes at 3000rpm. The supernatant was then decanted into a separate conical tube. The precipitate was washed by adding 40% ethanol at pH 6.0, mixed, placed on ice for at least 20 minutes or overnight in the freezer and then centrifuged as before. Finally, the precipitate was washed with Ultrapure water into a pre-weighed flask, frozen and dried by freeze-drying.

The supernatants from above were decanted into pre-weighed centrifugation bottles and centrifuged again at approx. 4000rpm for a minimum of 20 minutes. The supernatants were transferred into separate round bottom flask for rotary evaporation. The ethanol was removed from the supernatant by rotary evaporation whereby the temperature of the water bath did not exceed 35°C. Following removal of the ethanol, the aqueous supernatant was transferred to a pre-weighed flask, frozen and the water removed by freeze-drying.

The non-glycosylated and glycosylated AFP type III contents of the resulting freeze dried preparations are shown in Table 1.

**Table 1: AFP type III profiles of the freeze dried ethanol precipitate and supernatant.**

| Material | Non-glycosylated AFP type III as % total protein | Glycosylated AFP type III as % total protein |
|---|---|---|
| Freeze dried ethanol supernatant | 0.4 | 41 |
| Freeze dried ethanol precipitate | 39.5 | 19 |

Whilst the ethanol precipitate still contains some non-glycosylated material, the supernatant is highly enriched in glycosylated material (41% of total protein) compared to the non-glycosylated component (0.4% total protein).

### Recrystallisation inhibition (RI) assay

The recrystallisation inhibition activity of glycosylated HPLC 12 was used to determine the activity of the glycosylated and non-glycosylated AFP type III. A sample of 0.0004% protein in 30% sucrose solution was prepared and measured (3 repeats) in the RI assay. Two control samples were also measured: 30% sucrose solution (i.e. containing no AFP) and 0.0004% non-glycosylated HPLC 12. The results are presented as the change in the mean ice crystal size after undergoing recrystallisation at -6°C for 1 hour (Table 2).

**Table 2: RI inhibition results**

| Test solution | Growth (microns) |
|---|---|
| Control sucrose solution | 13.0 ± 0.5 |
| Non-glycosylated AFP type III HPLC 12 | 0.7 ± 0.5 |
| Glycosylated AFP type III HPLC 12 | 13.4 ± 0.5 |

The above results show that the non-glycosylated AFP type III HPLC-12 is active as it significantly reduces the amount of growth compared to the control sucrose solution. However, the glycosylated HPLC-12 shows the same growth as the sucrose solution. Therefore, the glycosylated AFP type III HPLC-12 has no effect on the recrystallisation, i.e. it is inactive.

### Example 2. Preparation of protein mannosyl transferase (pmt) deficient mutants

The pmt deficient mutants were constructed in *Saccharomyces cerevisiae* VWK18*gal1* (*MATa, leu2, gal1:URA3, ura3*) using the cre/lox gene disruption system described by Guldener et al (Nucleic Acids Res 24(13):2519-24, 1996). DNA fragments with short-flanking homology were generated by PCR using a loxP-KanloxP cassette. Correct integration of the cassette was verified by diagnostic PCR and subsequently the Kan gene was removed by expression of the cre recombinase. Correct removal of the cassette resulting in a deleted gene with one remaining loxP site was verified by diagnostic PCR.

The following deletions were constructed:
*pmt1*(201,2350)::loxP
*pmt2*(50,2229)::loxP
*pmt4*(09,2289)::loxP

### Example 3. Construction of mutant Saccharomyces strains transformed with a gene encoding AFP type III HPLC-12.

To construct a strain capable of efficient, controlled expression of AFP, *pmt* mutants of the host strain *S. cerevisiae* VWK18*gal1* were transformed with multiple copies of an ISP expression cassette derived from pUR3993 plasmid, designed to integrate at the rDNA locus as described by Lopes et al (Gene 1989 Jul 15;79(2):199-206). The rDNA integration cassette was excised from the complete plasmid by digestion with *HpaI* and the approximately 6283 bp fragment introduced into the host strain by trasformation using the lithium acetate method (Gietz R.D. and Woods R.A. Methods Enzymol 2002;350:87-96 ).

The detail of the rDNA integration cassette and the pUR3993 plasmid is shown in figures 1 (a) and (b) respectively. Transformants were selected for their ability to grow on minimal medium without leucine and screened for production of the AFP during growth on medium containing glucose as carbon source and galactose as inductor.

### Example 4. Determination of AFP III HPLC-12 content in fermentation samples

The sample components are separated by reverse phase HPLC using a C18 column and the AFP type III HPLC-12 content determined by UV detection at 214nm by reference to a purified standard.

### Apparatus:

AKTA Explorer XT 10
Analytical balance
Various glassware
Various pipettes (minimum class b)

### Reagents:

| | |
|---|---|
| Ultrapure water | Millipore water system |
| Acetonitrile | HPLC grade, Far UV |
| Trifluoroacetic acid (TFA) | HPLC grade |
| Isopropanol | HPLC grade |

### Preparation of eluents:

### Eluent A: 0.05% TFA in Ultrapure water

A volume of 1ml TFA was diluted to two litres with Ultrapure water and mixed.

### Preparation of eluent B: 0.05% TFA in acetonitrile

A volume of 0.5ml TFA was diluted to one litre with acetonitrile.

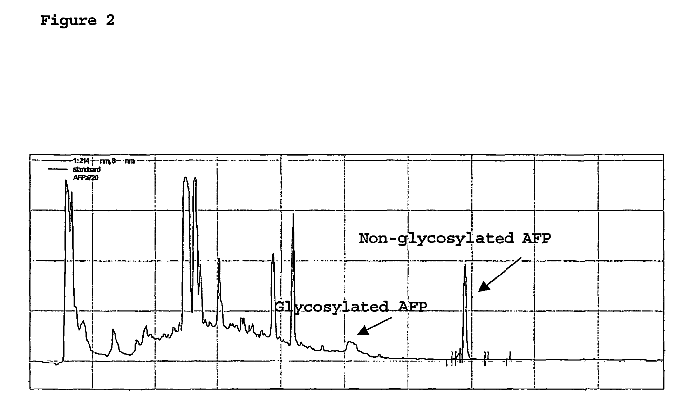

To prepare samples a volume or weight of test material was accurately pipetted/weighed, in triplicate, into separate 50ml volumetric flasks and made to volume with eluent A. Samples were filtered prior to being analysed using the below specified AKTA conditions. Purified non-glycosylated AFP type III was used as the quantification standard. A chromatograph from a typical fermentation sample is shown in figure 2.

The HPLC conditions used for AFP type III analysis were as follows:

| | | |
|---|---|---|
| Injection type | : | Partial fill |
| Injection loop | : | 100µl |
| Injection volume : | | 50µl |
| Needle wash | : | 20% IPA |
| Data handling | : | Compaq deskpro computer |
| | | Windows NT |
| | | UNICORN V3.21 software |
| | | UNICORN/A-900 software |
| Column | : | Vydac Protein/peptide C18 218TP54 |
| Mobile phase | : | A11, 0.05% TFA in Ultrapure water |
| line | | |
| | : | B1, 0.05% TFA in acetonitrile |
| Gradient | : | T0 → T5:100% A11 |
| | | T5 → T35:100% A11 → 42% All, 58% B1 |
| | | T36 → T40: 42% A11, 58% B → 100% B1 |
| | | T40 → T41.5: 100% B1 → 100% A11 |
| | | T41.5 → T44: 100% A11 |
| Flow rate | : | 1.0ml/min |

The AKTA Explorer 10XT chromatography system was fitted with A900 autosampler and a triple wavelength UV detector. Quantification was achieved using the 214nm signal. Other wavelengths such as 254 and 280nm were used for fingerprinting purpose only.

### Example 5. Effect of pmt deletion on AFP type III HPLC-12 production in laboratory scale fermenters.

Fermentations were carried out with each pmt mutant to determine the effect of the deletion on AFP production compared to the parent strain without any pmt deficiency. Fermentations were carried out as detailed below.

### Inoculum preparation

A shake flask containing 50 ml medium consisting of 6.7 g/l YNB (yeast nutrient broth) w/o amino acids (Difco) and 5 g/l glucose·1aq (Avebe) was inoculated with 1.4 ml glycerol stock of the strain and incubated during 48 hours at 30°C at 120 rpm. Subsequently, the inoculum was transferred to a shake flask containing 500 ml medium consisting of 10 g/l Yeast extract (Difco), 20 g/l Bacto Pepton (Difco) and 20 g/l glucose·1aq followed by incubation for 24 hours, 30°C at 120 rpm.

### Fed batch fermentations

The 5.5L batch medium consisted of 22 g/kg glucose·1aq, 10 g/kg yeast extract KatG (Ohly), 2.1 g/kg KH₂PO₄, 0.6 g/kg MgSO₄·7H₂O, 0.4 g/kg Struktol J673 (Schill & Seilacher), 10 g/kg Egli trace metals (a 100x solution of 5.5 g/l CaCl_{2·}2H₂O, 3.73 g/l FeSO₄·7H₂0, 1.4 g/l MnSO₄·1H₂O, 1.35 g/l ZnSO₄·7H₂O, 0.4 g/l CuSO₄·5H₂O, 0.45 g/l CoCl₂·6H₂O, 0.25 g/l NaMoO₄·2H₂O, 0.4 g/l H₃BO₃, 0.25 g/l KI, 30 g/l NaEDTA), 1 g/kg Egli vitamins (a 1000x solution of 5 g/l thiamin, 47 g/l meso-inosit, 1.2 g/l pyridoxin, 23 g/l panthotenic acid, 0.05 g/l biotin). The 4L feed medium contained 440 g/kg glucose·1aq, 3 g/l galactose (Duchefa), 25 g/kg yeast extract, 12 g/kg KH₂PO₄, 2.5 g/kg MgSO₄·7H₂O, 0.8 g/kg Struktol J673, 20 g/kg Egli trace metals, 2 g/kg Egli vitamins.

The fed batch fermentations were performed in standard bioreactors with a working volume of 10 litres. Dissolved oxygen (DO₂) was measured with an Ingold DO₂ electrode (Mettler-Toledo) and controlled by automatic adjustment of the speed of the 6-bladed Rushton impeller to a maximum of 1000 rpm. The pH was measured with an Ingold Impro 3100 gel electrode (Mettler-Toledo) and controlled using 3 M phosphoric acid (Baker) and 12.5 % v/v ammonia (Merck). Temperature was measured by a PT100 electrode and controlled via a cooling jacket and cooling and heating fingers.

The batch phase was started by transferring 500 ml of full grown inoculum to the batch medium. The temperature was maintained at 30°C and airflow at 2 l/min. DO₂ was controlled above 30%, pH at 5.0. When the ethanol signal in the off-gas decreased below 300 ppm the feed phase was started. In the feed phase the temperature was decreased to 21°C and the airflow was set to 6 l/min. The feed rate was applied according to an exponential profile required to maintain a growth rate of 0.06 1/h. The exponential feed continued until the DO₂ level in the fermenter decreased below 15% whereafter the feed rate was maintained linear.

**Table 3: Yields of glycosylated and non-glycosylated AFP type III HPLC-12 determined by reverse phase HPLC**

| Test organism | Total AFP normalised to parent strain productivity | Non-glycosylated AFP as % total | Fold increase in Non-glycosylated AFP production |
|---|---|---|---|
| parent strain | 1.0 | 23% | 1 |
| pmt1 mutant | 0.79 | 67% | 2.3 |
| pmt2 mutant | 0.61 | 71% | 1.9 |
| pmt4 mutant | 0.93 | 23% | 0.93 |

The yield of total AFP after 60 hrs fermentation and the effect of pmt deletion on non-glycosylated AFP productivity was determined using reverse phase HPLC and is shown in table 3.

The data in table 3 show that deletion of either pmt1 or pmt2 results in an increase in the % of non-glycosylated AFP produced compared to the parent strain. Although the total AFP yield is slightly decreased for both pmt1 and pmt2 mutants, the non-glycosylated yield is increased due to the decreased glycosylation activity resulting in a 2.3 fold and 1.9 fold overall increase in non-glycosylated AFP for pmt1 and pmt2 respectively. By contrast, deletion of pmt4 apparently has little or no effect on the % non-glycosylated product produced but does appear to slightly decrease the overall AFP yield. A comparison of the protein profiles for the parent strain and the pmt1 mutant on SDS gel showed that the original non-deficient strain contains both glycosylated and non-glycoslylated AFP whilst the pmt1 mutant produces predominantly non-glycosylated AFP (data not shown). Similar results were obtained from shake flask screening experiments.

This provides a relatively quick screening method for identifying strains with reduced ability to glycosylate the AFP protein.

### Example 6. Analysis of Glycosylation patterns of AFP type III HPLC-12 secreted by a transformed mutant strain

Investigation of the AFP type III glycoform patterns of the pmt1 mutant compared to the original non-deficient strain was performed by HPLC-MS. The degree of glycosylation and the relative abundance of AFP versus its main glycoforms (AFP with 5-13 mannose units) are compared using the selected ion monitoring (SIM) mass spectrometric responses of their respective most abundant protonated molecular ions. Detection is performed by positive electrospray ionisation mass spectrometry. Separation was achieved by gradient elution using a reversed phase HPLC column as described below.

### Apparatus & Reagents:

1050 HPLC module (Hewlett Packard)
Quattro I mass spectrometer (VG, now Micromass)
PRP1 column 4.6 x 250 mm (Hamilton)
Ultrapure water - Millipore-Q water system
Acetonitrile gradient HPLC grade

### Preparation of mobile phases for HPLC

A: 1% acetic acid in water
B: 1% acetic acid in 80% aq. acetonitrile

### Sample preparation

The samples were diluted 1 in 50 in water (1 g in 50 ml water) and filtered (0.45 µm or smaller syringe filter) prior to analysis.

### Equipment conditions

| HPLC system | | |
|---|---|---|
| UV detector | 214nm | |
| Injection volume | 20 µl (partial loop filling) | |
| Column | Phenomenex Jupiter C18 300A pore, 150 x 2.1 id mm | |
| Mobile phases | Maintained at 30°C | |
| | A: 1 % acetic acid in water | |
| Flow rate | B: 1 % acetic acid in 80% aq.acetonitrile | |
| Total analysis | 1 ml/min | |

| time | 74 minutes | |
|---|---|---|
| Gradient | Minutes | % B |
| | 0 | 10 |
| | 10 | 10 |
| | 55 | 65 |
| | 57 | 100 |
| | 62 | 100 |
| | 64 | 10 |
| | 74 | 10 |

A split rate of 1/5 was applied after the chromatographic separation to deliver 200 µl/min to the mass spectrometer

The QuattroI mass spectrometer

| | | |
|---|---|---|
| Tune page | Capillary | 3.2 V |
| settings | Cone | programmed as part of the |
| (file | HV Lens | method |
| | Source block temperature | 0.6 V |
| | Desolvation temperature | 150 C |
| | Multiplier | 150 C |
| | | 650 V |
| | Desolvation gas flow | |
| | Nebuliser gas flow | 300 l/h |
| | | 25 l/h |
| | | |
| MS method | Data is collected between 20 and 60 minutes | |
| | Scan: m/z 100 to 2000 (scan time: 5 sec, interscan | |
| | delay: 0.1 sec) | |
| | SIR function 1 (tracking of an AFP III fragment) | |
| | m/z 284.4 at cone voltage 70 V (marker for the last 3 amino acids at the C-terminal end of AFP III) and m/z 163.01 (oxonium ion, maker for glycopeptides) (span 1 Da, dwell time 0.08 sec, inter channel delay 0.02 sec) | |
| | SIR function 2 (ions for 6 charge state of AFP glycoforms) m/z 1308, 1335, 1362, 1389, 1416, 1443, 1470 and 1497 at cone voltage 20 V (span 1 Da, dwell time 0.08 sec, inter channel delay 0.02 sec). | |

Table 4 shows that the non-glycosylated AFP type III yield is increased from 23% to 67% using the pmt1 strain and that although the level of glycosylated product is reduced the glycosylation pattern is similar to that obtained from the non-deficient parent strain.

**Table 4**

| | **% of Total AFP type III HPLC12** | |
|---|---|---|
| | Parent | Pmt1 mutant |
| **Glycosylation type** | | |
| Unglycosylated | 25 | 67 |
| +5 Mannose | 4.5 | 2.2 |
| +6 Mannose | 10.5 | 4 |
| +7 Mannose | 11 | 5.5 |
| +8 Mannose | 13 | 5.8 |
| +9 Mannose | 12.5 | 5 |
| +10 Mannose | 9 | 3.2 |
| +11 Mannose | 8 | 4 |
| +12 Mannose | 6.5 | 3.3 |

Various modifications and variations of the described methods and system of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are apparent to those skilled in molecular biology or related fields are intended to be within the scope of the following claims.

### SEQUENCE LISTING PART OF THE DESCRIPTION

<210> 1
   <211> 66
   <212> PRT
   <213> Macrozoarces americanus
<400> 1 
Asn Gln Ala Ser Val Val Ala Asn Gln Leu Ile Pro Ile Asn Thr Ala
1               5                   10                  15

Leu Thr Leu Val Met Met Arg Ser Glu Val Val Thr Pro Val Gly Ile
            20                  25                  30

Pro Ala Glu Asp Ile Pro Arg Leu Val Ser Met Gln Val Asn Arg Ala
        35                  40                  45

Val Pro Leu Gly Thr Thr Leu Met Pro Asp Met Val Lys Gly Tyr Pro
    50                  55                  60

Pro Ala
65
 (Antifreeze protein type III HPLC-12 is specifically identified by accession number P19614 in the Swiss-Prot protein database)

## Claims

1. A method for producing a type III antifreeze protein (AFP) which method comprises expressing in a fungal host cell which is deficient in glycosylation of type III AFP in comparison to the parent strain, a nucleic acid sequence encoding the AFP, wherein the fungal cell is a yeast which is a pmt1- and/or pmt2-deficient mutant strain.

2. A method according to claim 1 wherein the yeast is *Saccharomyces cerevisiae.*

3. A method according to claim1 or claim 2 wherein the type III AFP is type III HPLC-12.

## Patentansprüche

1. Verfahren zur Herstellung eines Typ III-Antifreeze-Proteins (AFP), wobei das Verfahren umfasst Exprimieren in einer Pilzwirtszelle, die bezüglich Glycosylierung von Typ III-AFP im Vergleich zum Elternstamm defizient ist, einer Nucleinsäuresequenz, die für das AFP codiert, wobei die Pilzzelle eine Hefe ist, die ein pmt1- und/oder pmt2-defizienter Mutantenstamm ist.

2. Verfahren nach Anspruch 1, wobei die Hefe *Saccharomyces cerevisiae* ist.

3. Verfahren nach Anspruch 1 oder Anspruch 2, wobei das Typ III-AFP Typ III-HPLC-12 ist.

## Revendications

1. Méthode de production d'une protéine antigel (AFP, antifreeze protein) de type III, ladite méthode comprenant l'expression, dans une cellule hôte fongique se révélant déficiente en termes de glycosylation de l'AFP de type III par rapport à la souche mère, d'une séquence d'acide nucléique codant pour l'AFP, dans laquelle la cellule fongique est une levure de souche mutante présentant un déficit de pmt1 et/ou pmt2.

2. Méthode selon la revendication 1, dans laquelle la levure est *Saccharomyces cerevisiae.*

3. Méthode selon la revendication 1 ou la revendication 2, dans laquelle l'AFP de type III est l'HPLC-12 de type III.
